# EUROPEAN PATENT APPLICATION

(11) **EP 1 102 195 A2**
(43) Date of publication of application: **23.05.2001**
(21) Application number: 00307341.8
(22) Date of filing: 25.08.2000
(51) Int. Cl.: G06F 19/00

(54) **Method of providing welfare information**

(30) Priority: 17.11.1999 JP 32651099
(71) Applicant: Hitachi, Ltd., Chiyoda-ku, Tokyo (JP)
(72) Inventor: Wasaki, Yumiko, 5-1,Marunouchi 1-chome Chiyoda-ku Tokyo (JP)
(74) Representative: Hackney, Nigel John

(57) **Abstract**

A method of providing welfare information for accumulating and providing data on care plans and welfare personnel information, having a step to register care plans formulated by care managers, a step to register progress information on the recipient of care service provided in accordance with each of the care plans, a step to assess the effectiveness of each of the care plans according to the progress information and to rank the pertinent care manager, and a step to provide the resultant ranking as welfare personnel information.

## Description

The present invention relates to a welfare information providing system for providing welfare personnel information and care plan information, and more particularly to a technology that can be effectively applied to a welfare information providing system for accumulating welfare personnel information obtained by assessing and analyzing care plans and providing welfare personnel information care plan information as part of medical information providing service.

One of the already available types of service for providing information to customers is medical information providing service. This medical information providing service, besides responding to consultation from customers by e-mail, offers hospital searching service utilizing a medical institution information database (DB).

On the other hand, there is proposed a care insurance system under which an insured person is assured of care service when the person or another person close to him or her requires care service and the cost of care is equitably borne by the whole society so that the burden on caring persons can be minimized. This system is based on an institutionalized mechanism to enable each eligible recipient of care, officially recognized as such, to appropriately and effectively receive, when he or she actually is provided with care, within the coverage of the available benefit such service as fits his or her individual needs and conditions. The qualified recipient can request a specialist care manager not only to prepare a care plan to specify the types and extent of service but also to get in touch with a care service provider or the like and to introduce suitable facilities so that he or she can secure the supply of care service in accordance with the care plan so prepared.

Now that this care insurance system is about to be implemented, information providing service, such as the aforementioned medical information providing service, is required to provide the insured persons with information regarding the particulars of service available from welfare service providers and the outlines of facilities in addition to the aforementioned medical information. On the part of the welfare service provider, it is an urgent task to secure care managers who can coordinate proper services for each particular case of need for care and can prepare an appropriate care plan, and in order to accomplish this task it is essential to obtain without delay information on personnel having suitable expertise. As the market is still in an early stage of development, the criteria of evaluating a care manager tend to focus on whether he or she is officially qualified or not.

The Japanese Published Unexamined Patent Application No. Hei 11-53455 discloses a care service planning support apparatus, which can support care managers in the performance of their duties, compensate for the shortage of capable care managers, maintain the assessed capabilities of less capable care mangers at a constant level and, for more capable care managers, shorten the length of time needed for assessment.

The conventional information providing service referred to above, as it does not provide welfare personnel information revealing the whereabouts of care managers with expertise of higher standards, involves the problem that it is difficult to request a more capable care manager to prepare a care plan or to recruit care managers with expertise of higher standards, making it difficult to improve the quality of care service supplied to eligible recipients.

The conventional information providing service described above, as it provides no care plan information regarding different models or actual cases of care plans relevant to the circumstances of individual recipients, involves a further problem that a care plan is difficult to be worked out in some cases or the care plan cannot be customized to the needs of a given recipient.

The conventional information providing service mentioned above, as it quantitatively measures the level of achievement of service provision as initially planned and the level of the recipient's satisfaction with the provided service, which is a subjective measure of the recipient, and excludes service providers who are ranked low in the levels of achievement and/or of recipient satisfaction thereby to enhance the quality and recipient satisfaction of the service, involves another problem that it is difficult to objectively assess whether or not the contents of the care plan were appropriate.

Preferably an object of the present invention is to provide a technology that solves the above-noted problems.

Preferably another object of the invention is to provide a technology that makes possible supporting of the formulation of care plans.

According to an aspect of the invention, there is provided a welfare information providing system for accumulating and providing data regarding care plans and welfare personnel information, the welfare personnel information comprising the ranking of care managers obtained by assessment of the effectiveness of care plans according to information on the progress of recuperation of care service recipients. Preferably in the welfare information providing system according to the invention, a care plan formulated by a care manager and information on the progress of recuperation of a recipient of the care service according to the care plan are first registered into the care plan DB of a welfare data bank apparatus.
preferably, next, the effectiveness of the care plan is assessed according to the progress information, the care manager is ranked on that basis and registered into a care manager DB, and the resultant ranking is provided to a welfare service provider's apparatus as welfare personnel information.

The welfare information providing system according to the invention, as it assesses the effectiveness of the care plan according to the progress information on the recipient and provides welfare personnel information, makes it possible to improve the quality of care service provided to its eligible recipients.

### BRIEF DESCRIPTION OF THE DRAWINGS

Other objects and advantages of the invention will become apparent during the following discussion of the accompanying drawings, wherein:
Fig. 1 schematically illustrates the configuration of a welfare information providing system, which is a preferred embodiment of the present invention;
Fig. 2 schematically illustrates the configuration of a care manager's apparatus 102 in this embodiment of the invention;
Fig. 3 schematically illustrates the configuration of a welfare data bank apparatus 103 in this embodiment of the invention;
Fig. 4 schematically illustrates the configuration of a welfare service provider's apparatus 104 in this embodiment of the invention;
Fig. 5 is a flowchart showing the processing sequence of care plan registration in this embodiment of the invention;
Fig. 6 illustrates typical contents of registered care plan information in this embodiment of the invention;
Fig. 7 is a flowchart showing the processing sequence of progress information registration in this embodiment of the invention;
Fig. 8 illustrates typical contents of registered progress information in this embodiment of the invention;
Fig. 9 is a flowchart showing the processing sequence of care plan assessment in this embodiment of the invention;
Fig. 10 illustrates an example of care plan assessment in this embodiment of the invention;
Fig. 11 is a flowchart showing the processing sequence of providing welfare personnel information in this embodiment of the invention; and
Fig. 12 is a flowchart showing the processing sequence of providing a care plan in this embodiment of the invention.

### DETAILED DESCRIPTION OF PREFERRED EMBODIMENTS

A welfare information providing system, which is a preferred embodiment of the invention, for accumulating and providing data regarding care plans and welfare personnel information will be described below.

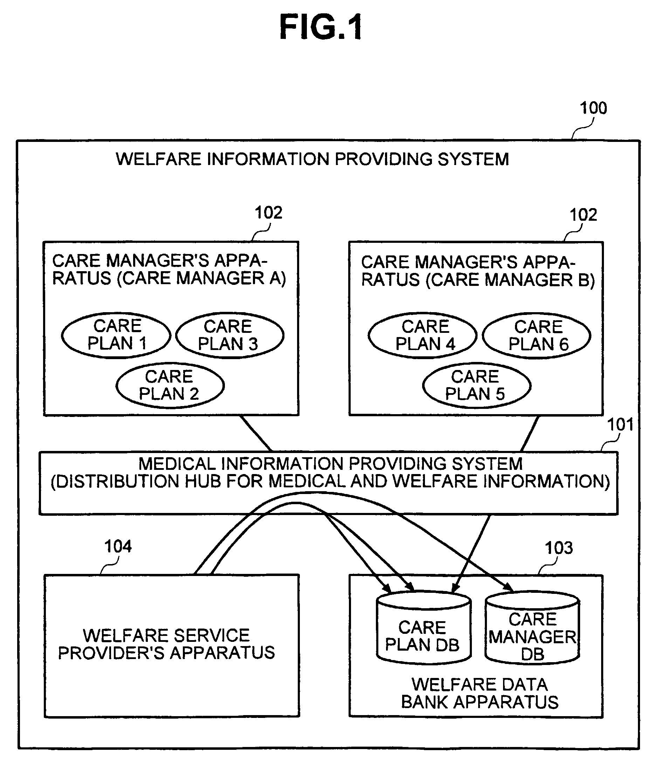

Fig. 1 schematically illustrates the configuration of the welfare information providing system. As shown in Fig. 1, a welfare information providing system 100, which is the embodiment of the invention, has a medical information providing system 101, care manager's apparatuses 102, a welfare data bank apparatus 103 and a welfare service provider's apparatus 104.

The medical information providing system 101 is a system to connect the care manager's apparatuses 102 and the welfare service provider's apparatus 104 to the welfare data bank apparatus 103 and thereby to relay care plans, welfare personnel information and so forth. The care manager's apparatuses 102 are apparatuses on the care managers' side for requesting registration of care plans formulated by care managers and progress information on care service recipients and referencing similar examples of care plans.

The welfare data bank apparatus 103 is an apparatus for registering care plans and progress information on care service recipients from the care manager's apparatuses 102 and ranking care managers, the resultant ranking being provided as welfare personnel information. The welfare service provider's apparatus 104 is an apparatus on the part of a care service provider for referencing welfare personnel information and similar examples of care plans registered in the welfare data bank apparatus 103.

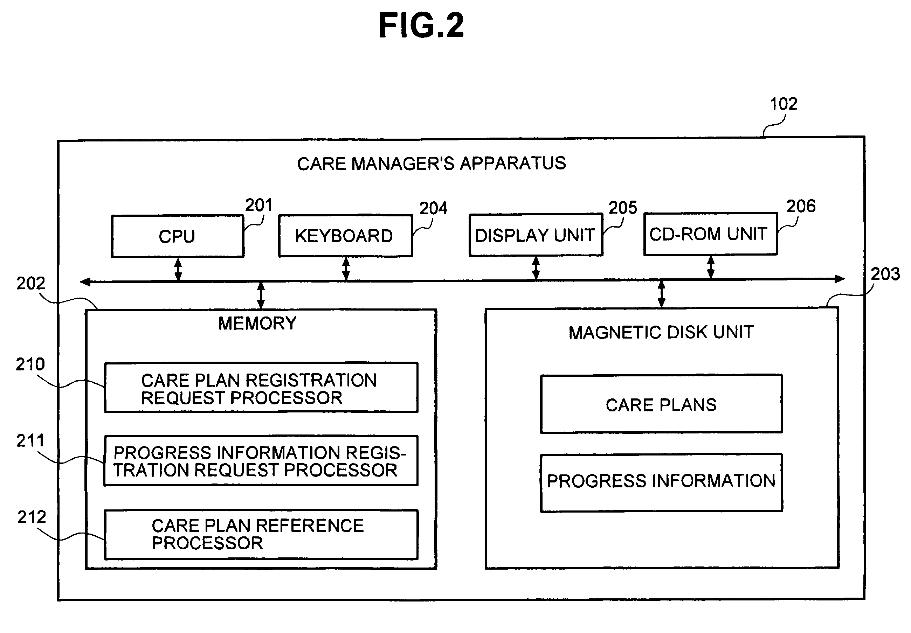

Fig. 2 schematically illustrates the configuration of a care manager's apparatus 102 in this embodiment of the invention. As shown in Fig. 2, a care manager's apparatus 102 in this embodiment of the invention has a CPU 201, a memory 202, a magnetic disk unit 203, a keyboard 204, a display unit 205 and a CD-ROM unit 206.

The CPU 201 is a unit for performing overall control on the care manager's apparatus 102. The memory 202 is a memory into which, when the overall operation of the care manager's apparatus 102 is to be controlled, various processing programs and data for use in the control are to be loaded.

The magnetic disk unit 203 is a memory for storing data, such as the above-mentioned various processing programs and care plans. The keyboard 204 is a unit for entering requests for the registration of care plans and progress information and executive instructions, such as ones to reference care plans.

The display unit 205 is a unit for displaying a message or the like for requesting the registration of a care plan or progress information or referencing care plans. The CD-ROM unit 206 is a unit for reading the contents of a CD-ROM on which the aforementioned various programs are recorded. A care manager's apparatus 102 further has a care plan registration request processor 210, a progress information registration request processor 211 and a care plan reference processor 212.

The care plan registration request processor 210 is a processor for requesting the welfare data bank apparatus 103 to register a care plan formulated by the care manager. The progress information registration request processor 211 is a processor for requesting the welfare data bank apparatus 103 to register progress information on the care service recipient to whom care service has been provided in accordance with the care plan.

The care plan reference processor 212 is a processor for referencing similar examples among care plans registered in the welfare data bank apparatus 103 when, for instance, a case for which it is difficult to formulate a care plan is encountered.

A program to enable the care manager's apparatus 102 to function as the care plan registration request processor 210, the progress information registration request processor 211 and the care plan reference processor 212 is, after being recorded onto a recording medium such as a CD-ROM and stored onto a magnetic disk or the like, loaded into a memory to be executed. The recording medium to record the program may be something else than a CD-ROM.

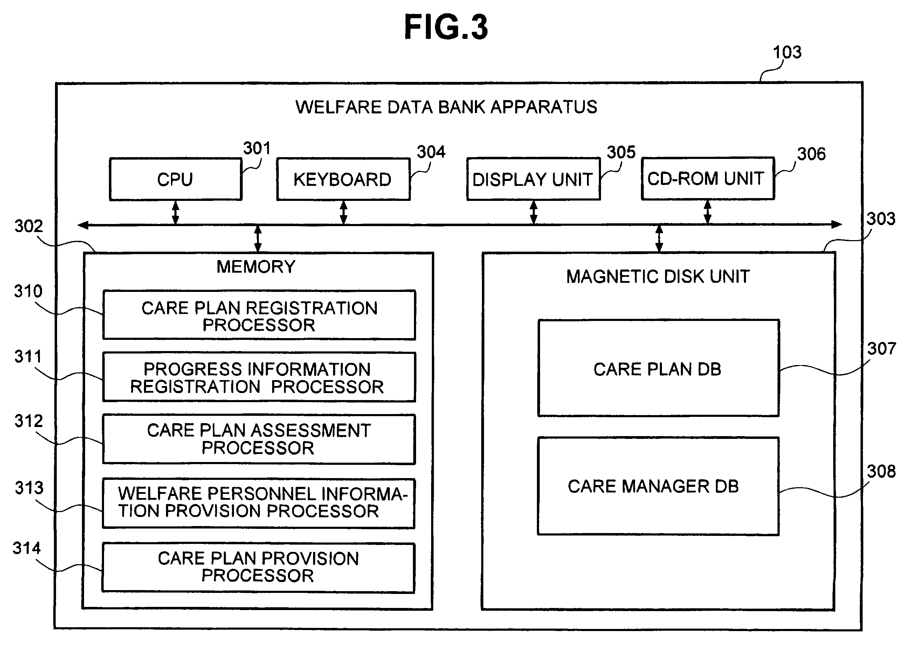

Fig. 3 schematically illustrates the configuration of the welfare data bank apparatus 103 in this embodiment of the invention. As shown in Fig. 3, the welfare data bank apparatus 103 in this embodiment of the invention has a CPU 301, a memory 302, a magnetic disk unit 303, a keyboard 304, a display unit 305, a CD-ROM unit 306, a care plan DB 307 and a care manager DB 308.

The CPU 301 is a unit for performing overall control on the welfare data bank apparatus 103. The memory 302 is a memory into which, when the overall operation of the welfare data bank apparatus 103 is to be controlled, various processing programs and data for use in the control are to be loaded.

The magnetic disk unit 303 is a memory for storing data, such as the above-mentioned various processing programs, care plans and welfare personnel information. The keyboard 304 is a unit for entering operational instructions for the registration of care plans and progress information and the supply of welfare personnel information and care plans.

The display unit 305 is a unit for displaying a message or the like for the registration of care plans and progress information and the supply of welfare personnel information and care plans. The CD-ROM unit 306 is a unit for reading the contents of a CD-ROM on which the aforementioned various programs are recorded.

The care plan DB 307 is a database in which care plans formulated by care managers and progress information on the recipients of care service according to the care plans are registered. The care manager DB 308 is a database in which the ranking of care managers obtained by the assessment of the effectiveness of care plans according to the progress information is registered as welfare personnel information.

The welfare data bank apparatus 103 has a care plan registration processor 310, a progress information registration processor 311, a care plan assessment processor 312, a welfare personnel information provision processor 313 and a care plan provision processor 314.

The care plan registration processor 310 is a processor for receiving from a care manager's apparatus 102 care plans formulated by the care manager, registering them into the care plan DB 307, classifying the care plans according to the findings of case studies on recipients, and storing the classified care plans.

The progress information registration processor 311 is a processor for receiving progress information on the recipients of care service according to the care plans from the care manager's apparatus 102, each recipient, the recipient's family or the like and registering it into the care plan DB 307.

The care plan assessment processor 312 is a processor assessing the effectiveness of a given care plan according to the progress information, ranks the pertinent care manager and registers the ranking into the care manager DB 308. The welfare personnel information provision processor 313 is a processor for providing the ranking to the welfare service provider's apparatus 104 as welfare personnel information.

The care plan provision processor 314 is a processor for providing the care plans classified according to the findings of case studies, after taking steps to protect private information on recipients in the care plans, to the care manager's apparatuses 102 and the welfare service provider's apparatus 104 as models of care plans or reference cases.

A program to enable the welfare data bank apparatus 103 to function as the care plan registration processor 310, the progress information registration processor 311, the care plan assessment processor 312, the welfare personnel information provision processor 313 and the care plan provision processor 314 is, after being recorded onto a recording medium such as a CD-ROM and stored onto a magnetic disk or the like, loaded into a memory to be executed. The recording medium to record the program may be something else than a CD-ROM.

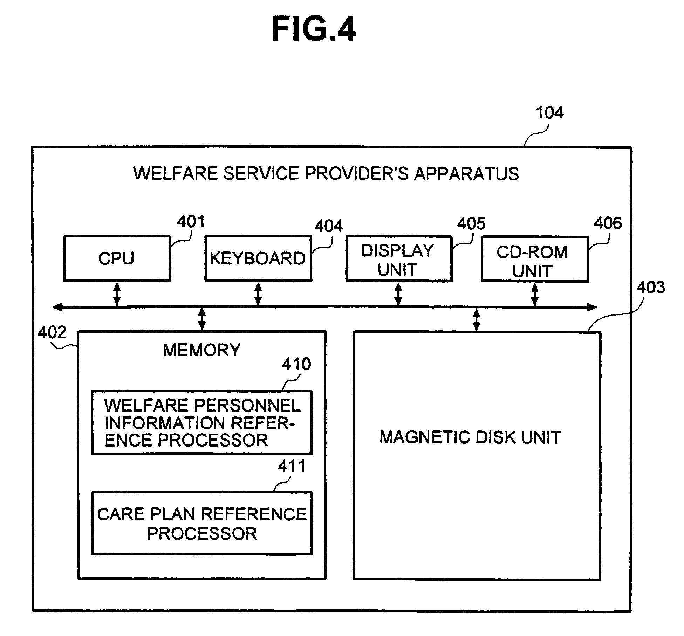

Fig. 4 schematically illustrates the configuration of the welfare service provider's apparatus 104 in this embodiment of the invention. As shown in Fig. 4, the welfare service provider's apparatus 104 in this embodiment of the invention has a CPU 401, a memory 402, a magnetic disk unit 403, a keyboard 404, a display unit 405 and a CD-ROM unit 406.

The CPU 401 is a unit for performing overall control on the welfare service provider's apparatus 104. The memory 402 is a memory into which, when the overall operation of the welfare service provider's apparatus 104 is to be controlled, various processing programs and data for use in the control are to be loaded.

The magnetic disk unit 403 is a memory for storing data, such as the above-mentioned various processing programs and data. The keyboard 404 is a unit for entering operational instructions for referencing welfare personnel information and care plans or the like. The display unit 405 is a unit for displaying a message or the like for referencing welfare personnel information and care plans. The CD-ROM unit 406 is a unit for reading the contents of a CD-ROM on which the aforementioned various programs are recorded.

The welfare service provider's apparatus 104 has a welfare personnel information reference processor 410 and a care plan reference processor 411.

The welfare personnel information reference processor 410 is a processor for referencing welfare personnel information registered in the welfare data bank apparatus 103 and displaying information on personnel having superior expertise.

The care plan reference processor 411 is a processor for referencing similar examples among care plans registered in the welfare data bank apparatus 103 when, for instance, a case for which it is difficult to formulate a care plan is encountered.

A program to enable the welfare service provider's apparatus 104 as the welfare personnel information reference processor 410 and the care plan reference processor 411 is, after being recorded onto a recording medium such as a CD-ROM and stored onto a magnetic disk or the like, loaded into a memory to be executed. The recording medium to record the program may be something else than a CD-ROM.

The procedure of processing by the welfare information providing system 100 in this embodiment of the present invention will be described below.

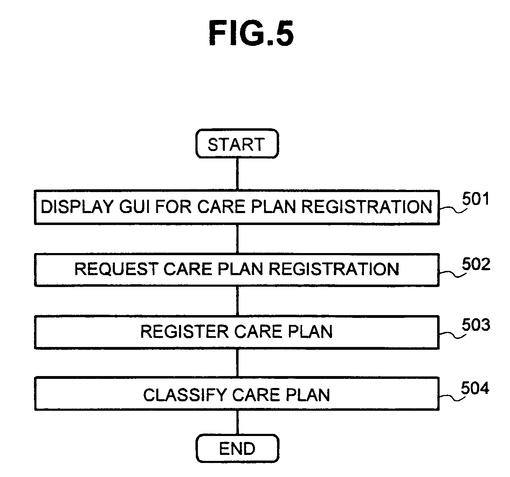

Fig. 5 is a flowchart showing the processing sequence of care plan registration in this embodiment of the invention. Fig. 5 covers the steps of processing from the receipt of a care plan formulated by a care manager from a care manager's apparatus 102 to its registration into the care plan DB 307 of the welfare data bank apparatus 103.

At step 501, the care plan registration request processor 210 of the care manager's apparatus 102 displays a graphical user interface (GUI) for supporting the registration of the care plan. The care manager determines the particulars of the care service to be provided to the pertinent recipient, enters them into the care manager's apparatus 102 as care plan information, and instructs registration of the care plan.

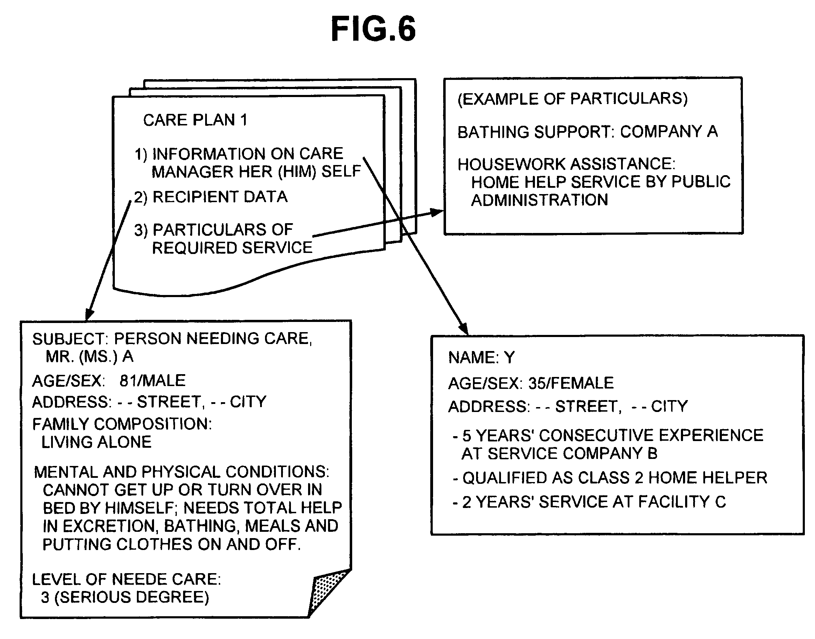

Fig. 6 illustrates typical contents of registered care plan information in this embodiment of the invention.

As shown in Fig. 6, care plan information registered by a care manager into the welfare data bank apparatus 103 includes (1) information on the care manager himself or herself, similar to a usual curriculum vitae containing the care manager's occupational history and so forth, (2) information on the recipient himself or herself and (3) the particulars of the formulated care plan

At step 502, the care plan registration request processor 210 is connected to the medical information providing system 101, and requests the welfare data bank apparatus 103 to register the entered care plan by sending an instruction to register the care plan to the welfare data bank apparatus 103 via the medical information providing system 101.

At step 503, the care plan registration processor 310 of the welfare data bank apparatus 103 receives from the care manager's apparatus 102 via the medical information providing system 101 the care plan information sent from the care manager, and registers it into the care plan DB 307 of the magnetic disk unit 303.

Next at step 504, the care plan registration processor 310 classifies the care plan registered into the care plan DB 307 according to the findings of case studies on recipients. The care plan is classified by, for instance, the recipient's age, sex, family composition and residential district; the level of needed care as to turning over in bed, movement, meals, excretion, putting clothes on and off, and bathing to set various cases according to the different criteria; and the care plan is classified by each such criterion for cases.

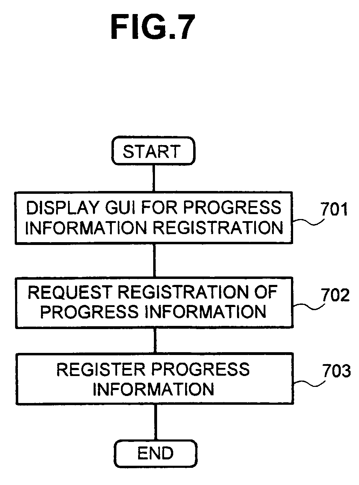

Fig. 7 is a flowchart showing the processing sequence of progress information registration in this embodiment of the invention. Fig. 7 covers the steps of processing from the receipt of progress information on the recipient of the care service in accordance with the care plan from the care manager's apparatus 102 to its registration into the care plan DB 307 of the welfare data bank apparatus 103. The progress information on the recipient of care service may as well be received from the recipient himself or herself or the recipient's family.

At step 701, the progress information registration request processor 211 of the care manager's apparatus 102 displays a GUI for supporting the registration of the progress information. The care manager enters into the care manager's apparatus 102 the progress information on the recipient of the care service in accordance with the care plan he or she formulated, and gives an instruction to register the progress information.

At step 702, the progress information registration request processor 211 is connected to the medical information providing system 101, and requests the welfare data bank apparatus 103 to register the entered progress information by sending an instruction to register the progress information to the welfare data bank apparatus 103 via the medical information providing system 101.

At step 703, the progress information registration processor 311 of the welfare data bank apparatus 103 receives the progress information on the care service recipient from the care manager's apparatus 102 via the medical information providing system 101, and registers it into the care plan DB 307 of the magnetic disk unit 303.

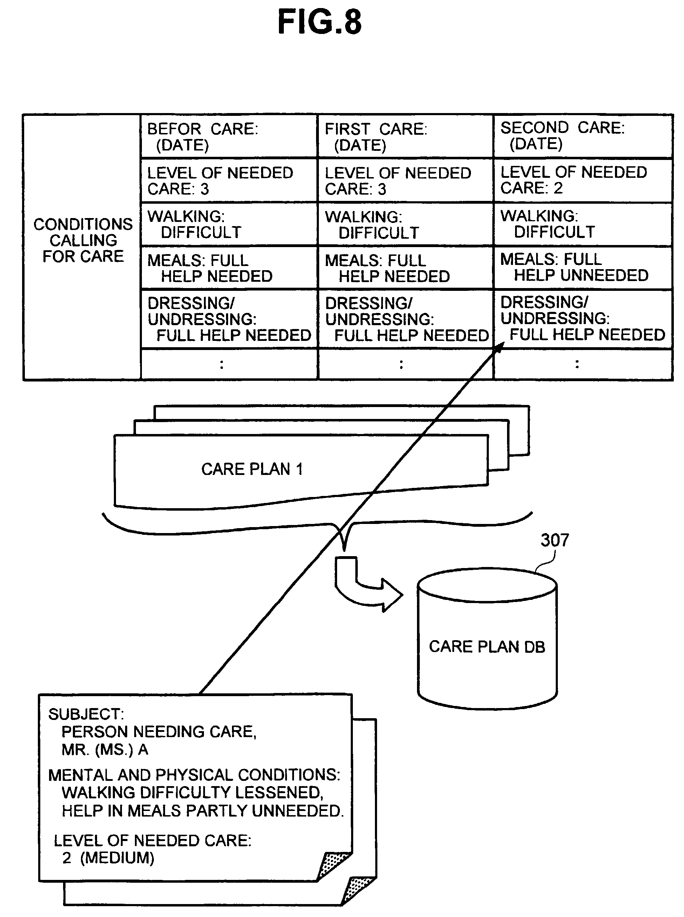

Fig. 8 illustrates typical contents of registered progress information in this embodiment of the invention. As shown in Fig. 8, the progress information which the care manager registers into the care plan DB 307 of the welfare data bank apparatus 103, as information on the subsequent progress of the recipient of the recipient of care service in accordance with the care plan formulated by the care manager, contains information on the recipient's mental and physical conditions including his or her walking ability and degree of help received in meals and the level of needed care. The progress information may also include evaluation of the care plan by the recipient himself or herself and the recipient's family.

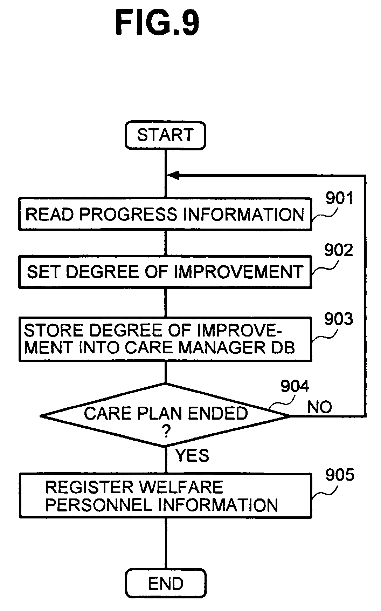

Fig. 9 is a flowchart showing the processing sequence of care plan assessment in this embodiment of the invention.

Fig. 9 covers the steps of processing from the assessment of the effectiveness of the care plan according to progress information to the ranking of the care manager and the registration of its result into the care manager DB 308.

At step 901, the care plan assessment processor 312 of the welfare data bank apparatus 103 references a care plan stored in the care plan DB 307 of the magnetic disk unit 303, and reads out of the care plan DB 307 the mental and physical conditions, before the start of care service, of the recipient of the care service in accordance with the care plan and information on the subsequent progress of his or her recuperation.

At step 902, the pre-care mental and physical conditions of the recipient and the information on the subsequent progress read out at step 901 are compared with each other for each item of progress information, and the degree of improvement thereby determined regarding each item of progress information is set, and stored into the care plan DB 307. For instance, comparison is made of each item of progress information, and if the mental and/or physical conditions are found improved, a degree of improvement "+1" is set, if no change is observed "0" is set, or if the conditions are found worsened, "-1" is set.

At step 903, the total degree of improvement of the care manager having formulated the care plan is read out of the care manager DB 308; the degrees of improvement in the individual progress information items which were set as described above are added to it; , and the sum is stored into the care manager DB 308. Although the total degree of improvement of the care manager in the care manager DB 308 is supposed to be initialized at the beginning of processing, a cumulative value may as well be used instead of initialization at the beginning of processing.

At step 904, it is checked whether or not the degree of improvement has been set with respect to progress information on every care plan, and if it has not yet been, the process returns to step 901 to perform processing to assess the next care plan or, if it has been, the process goes ahead to step 905.

At step 905, the total degrees of improvement stored in the care manager DB 308 of the magnetic disk unit 303 are referenced to rank the individual care managers, and the ranking is registered into the care manager DB 308 as welfare personnel information.

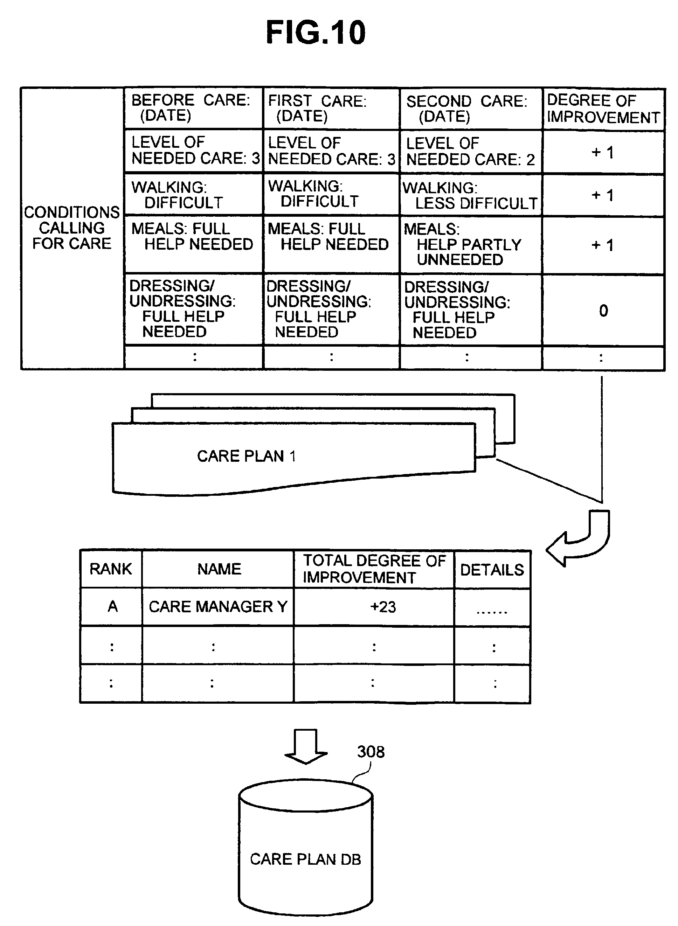

Fig. 10 illustrates an example of care plan assessment in this embodiment of the invention. As shown in Fig. 10, degrees of improvement are set for the progress information registered into the care plan DB 307 of the welfare data bank apparatus 103; the individual care managers are ranked on the basis of the total degree of improvement of the care manger having formulated each care plan; and the ranking is registered into the care manager DB 308 as welfare personnel information.

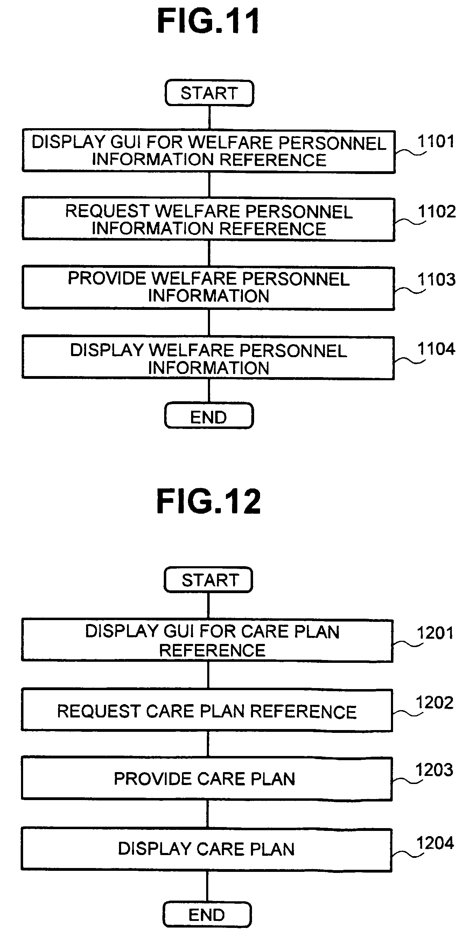

Fig. 11 is a flowchart showing the processing sequence of providing welfare personnel information in this embodiment of the invention.

Fig. 11 shows the processing of referencing information on care managers registered in the welfare data bank apparatus 103, which is performed when the welfare service provider appoints a care manager or on a similar occasion.

At step 1101, the welfare personnel information reference processor 410 of the welfare service provider's apparatus 104 displays a GUI for supporting the processing to reference welfare personnel information. The manager of the welfare service provider enters into the welfare service provider's apparatus 104 the age, years of experience, geographical area and so forth as keywords relevant to the welfare personnel information to be referenced, and gives an instruction to reference pertinent welfare personnel information.

At step 1102, the welfare personnel information reference processor 410 is connected to the medical information providing system 101, and requests the welfare data bank apparatus 103 to reference welfare personnel information by sending the entered instruction to reference welfare personnel information to the welfare data bank apparatus 103 via the medical information providing system 101.

At step 1103, the welfare personnel information provision processor 313 of the welfare data bank apparatus 103 searches the care manager DB 308 using the relevant keywords sent from the welfare service provider's apparatus 104, sorts the results of search in the descending order of ranking, and provides the sorted results to the welfare service provider's apparatus 104 as welfare personnel information.

At step 1104, the welfare personnel information reference processor 410 of the welfare service provider's apparatus 104 displays on the display unit 305 the welfare personnel information provided by the welfare data bank apparatus 103. The manager of the welfare service provider references the welfare personnel information displayed on the display unit 305, and references it for recruiting personnel having superior expertise.

Fig. 12 is a flowchart showing the processing sequence of providing a care plan in this embodiment of the invention. Fig. 12 shows the processing by a care manager to reference similar examples among care plans registered in the welfare data bank apparatus 103 when a case for which it is difficult to formulate a care plan is encountered. Alternatively, someone else than the care manager may reference care plans. For instance, the welfare service provider may do so by using the care plan reference processor 411 of the welfare service provider's apparatus 104 .

At step 1201, the care plan reference processor 212 of the care manager's apparatus 102 displays a GUI for supporting the processing to reference care plans. The care manager enters into the care manager's apparatus 102 keywords relevant to the care plan for which reference is to be made, and gives an instruction to reference care plans. The care manager can use as the relevant keyword, for instance, the pertinent case number assigned, when the care plan was registered into the care plan DB 307, to each of the cases classified according to the recipient's age, sex, family composition, residential district and the level of needed care, and as the relevant keywords specifically the recipient's age, sex, family composition, residential district and the level of needed care.

At step 1202, the care plan reference processor 212 is connected to the medical information providing system 101, and requests the welfare data bank apparatus 103 to reference care plans by sending the entered instruction to reference care plans to the welfare data bank apparatus 103 via the medical information providing system 101.

At step 1203, the care plan provision processor 314 of the welfare data bank apparatus 103 searches the care plan DB 307 using relevant keywords sent from the welfare service provider's apparatus 104; sorts the results of search in the descending order of the total level of improvement for each care plan; and provides the sorted results to the care manager's apparatus 102.

At step 1204, the care plan reference processor 212 of the care manager's apparatus 102 displays on the display unit 205 the care plan provided by the welfare data bank apparatus 103. The care manager references the care plan displayed on the display unit 205, and uses it as an example of care plan to reference when, for instance, examples similar to a case for which it is difficult to formulate a care plan are to be studied.

Hereupon, the care plan provision processor 314 of the welfare data bank apparatus 103 carries out masking to obscure private information in the care plan, including the recipient's name and address, to prevent those items of private information to be displayed on the display unit 205 of the care manager's apparatus 102 and thereby to protect private information on the recipient contained in the care plan. Private information on the recipient may as well be protected by the care plan reference processor 212 of the care manager's apparatus 102.

As described above, since this embodiment of the invention assesses the effectiveness of a given care plan according to progress information on care service recipients and provides welfare personnel information on that basis, it is made possible to assess the appropriateness of the particulars of the care plan objectively, and a welfare service provider is enabled to provide care service of higher quality than those provided by its competitors by referencing welfare personnel information and recruiting personnel superior in expertise.

Further, the welfare data bank apparatus 103 can accumulate patterns of conceivably optimal care plans according to the classification of case studies on recipients and build up a database of particulars of care plans as such, and the contents of the care plan DB 307 can be offered, through searching by keywords, as models of care plans or reference cases to care managers and other parties concerned.

A care manager can, by registering the care plans he or she has formulated into the welfare data bank apparatus 103, make his or her expertise known to welfare service providers and other parties concerned or, where a case for which it is difficult to formulate a care plan is encountered, search the care plan DB 307 for similar cases.

As hitherto described, since the welfare information providing system according to this embodiment of the invention assesses the effectiveness of a given care plan according to progress information on care service recipients and provides welfare personnel information on that basis, it is made possible to improve the quality of care service provided to recipients.

Furthermore, since the welfare information providing system according to this embodiment of the invention offers care plans according to the classification of case studies as models of care plans or reference cases, it can support formulation of care plans.

According to the present invention, since the effectiveness of a given care plan is assessed according to progress information on care service recipients and welfare personnel information is provided on that basis, it is made possible to improve the quality of care service provided to recipients.

## Claims

1. A method of providing welfare information for accumulating and providing data on care plans and welfare personnel information, comprising:
a step to register care plans formulated by care managers;
a step to register progress information on the recipient of care service provided in accordance with each of the care plans;
a step to assess the effectiveness of each of the care plans according to the progress information and to rank the pertinent care manager; and
a step to provide the resultant ranking as welfare personnel information.

2. A method of providing welfare information for accumulating and providing data on care plans and welfare personnel information, comprising:
a step to register care plans formulated by care managers and storing the care plans as classified according to the findings of case studies; and
a step to provide the care plans classified according to the findings of case studies to a care manager or a welfare service provider as models of care plans or reference cases.

3. A method of providing welfare information, as claimed in Claim 1, having a step to protect private information on the respective recipients of care service contained in the care plans.

4. A method of providing welfare information, as claimed in Claim 2, having a step to protect private information on the respective recipients of care service contained in the care plans.

5. A welfare information providing system for accumulating and providing data on care plans and welfare personnel information, comprising:
a care plan registration processor for registering care plans formulated by care managers;
a progress information registration processor for registering progress information on the recipient of care service provided in accordance with each of the care plans;
a care plan assessment processor for assessing the effectiveness of each of the care plans according to the progress information and ranking the pertinent care manager; and
a welfare personnel information provision processor for providing the resultant ranking as welfare personnel information.

6. A computer-readable recording medium recording thereon a program for causing a computer to function as a welfare information providing system for accumulating and providing data on care plans or welfare personnel information, said program causing a computer to function as:
a care plan registration processor for registering care plans formulated by care managers;
a progress information registration processor for registering progress information on the recipient of care service provided in accordance with each of the care plans;
a care plan assessment processor for assessing the effectiveness of each of the care plans according to the progress information and ranking the pertinent care manager; and
a welfare personnel information provision processor for providing the resultant ranking as welfare personnel information.
